# EUROPEAN PATENT SPECIFICATION

(11) **EP 1 674 052 B1**
(45) Date of publication and mention of the grant of the patent: **11.06.2008**
(21) Application number: 05256324.4
(22) Date of filing: 11.10.2005
(51) Int. Cl.: A61F 2/38

(54) **Knee prosthesis**
Knieprothese
Prothèse de genou

(30) Priority: 23.12.2004 US 19619
(43) Date of publication of application: 28.06.2006
(73) Proprietor: Corin Limited, Cirencester Gloucestershire GL7 1YJ (GB)
(72) Inventor: Gross, Thomas Paul, Columbia, SC 29223 (US)
(74) Representative: Bailey, Richard Alan

(56) References cited:
- WO-A-03/065939
- FR-A- 2 716 618
- US-A- 5 489 311
- US-A- 5 609 641
- US-A1- 2003 204 263
- US-A1- 2004 019 384
- US-B1- 6 296 667

## Description

The present invention relates to a tibial component for a knee prosthesis.

It is known from US6258127B1 to provide a tibial component, which has a tibial platform and an anchoring element in the form of a plate or shield. The anchoring element is flat except for the provision of screw receiving holes to enable connection of the tibial platform to the anchoring plate.

An initial problem associated with the device of US6258127B1 is that the distal edge of the anchoring plate is a cutting edge, intended to allow the plate to be forcibly driven into the prepared capsule of the tibia. Splitting of the tibia could therefore occur during implantation if great care is not taken by the surgeon.

Further problems associated with all known tibial components are the difficulty of removal of both the tibial platform and the stem or keel when, for example, a revision is required. The tibial platform is typically removed by sawing the platform from the stem or anchoring element, resulting in titanium or cobalt-chromium alloy shards and swarf being undesirably introduced into the body during the surgical procedure.

Once the tibial platform is finally removed, it can be extremely difficult and time consuming to then remove the stem or keel, often resulting in substantial damage to the surrounding bone of the tibia. Even when utilising a shield-type anchoring element in an arrangement such as suggested in US6258127B1, it is extremely problematic to form cuts on opposite sides of the anchoring element, which intersect and thus allow the anchoring element to be simply lifted out of the tibia.

It is known from FR2716618A, WO03065939A1, US20030204263A1, US5489311B, US5609641B, and US20040019384A to provide a tibial platform and a disengagble wedge-shaped keel. Such wedge-shaped keels are generally used to act as a strengthening means for the fixation stem. However, due to their relatively small surface area, fixation is often not satisfactory, and, although a space does exist between the inferior surface of the tibial platform and the upper surface of these keels, the space was not intended, and is therefore not sufficient, to receive a surgical cutting device therebetween.

The present invention seeks to overcome these problems.

According to the present invention, there is provided a tibial component for a knee prosthesis as defined in claim 1.

The present invention will now be more particularly described, by way of example only, with reference to the accompanying drawings, wherein :
Figure 1 is a rear posterior view of a first embodiment of a tibial component for a knee prosthesis, in accordance with the first, third and fourth aspects of the present invention;
Figure 2 is a side view of the tibial component shown in Figure 1;
Figure 3 is a rear view of a tibial platform of the tibial component shown in Figure 1;
Figure 4 is a top plan view of the tibial platform;
Figure 5 is a bottom plan view of the tibial platform;
Figure 6 is a perspective view from below of a keel of the tibial component shown in Figure 1;
Figure 7 is a top plan view of the keel;
Figure 8 is a front anterior view of the keel;
Figure 9 is a side view of the keel;
Figure 10 is a view similar to Figure 1 of a second embodiment of a tibial component, in accordance with the first, third and fourth aspects of the present invention;
Figure 11 is a sectional view of the tibial component shown in Figure 10, along the line A-A; and
Figure 12 is a perspective view from below of a third embodiment of a tibial component, in accordance with the first, third and fourth aspects of the present invention.

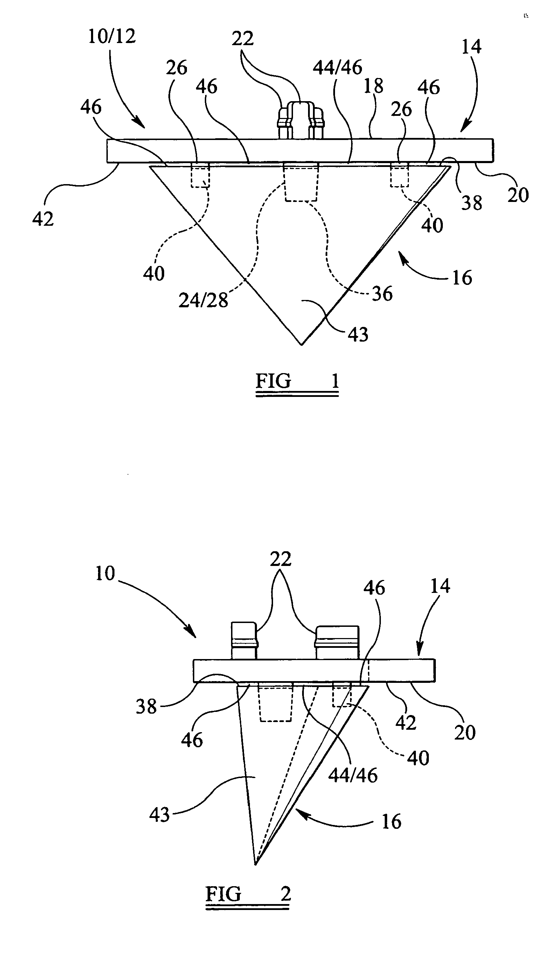

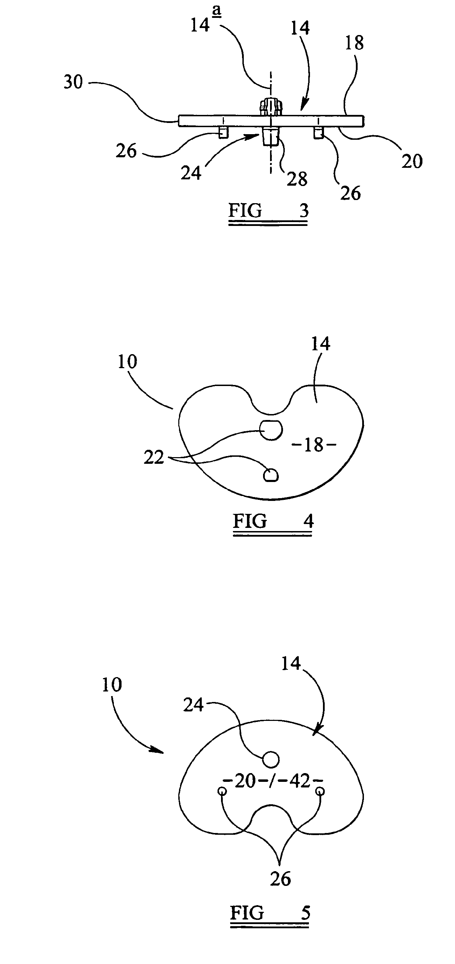

Referring firstly to Figures 1 to 9 of the drawings, there is shown a tibial component 10 for a knee prosthesis 12. The tibial component 10 is formed of a suitable biocompatible material, and comprises a tibial platform 14 and a keel 16. The tibial platform 14 and keel 16 are preferably formed from titanium alloy, cobalt-chromium alloy or other suitable biocompatible material. The tibial platform 14 has a superior surface 18 and an inferior surface 20.

The superior surface 18 of the tibial platform 14 includes two upstanding bollards 22, similar in design and function to those described in GB2345446A, for cooperation with a totally mobile meniscal component (not shown) of the knee prosthesis 12. However, the superior surface 18 of the tibial platform 14 can include any suitable arrangement for cooperation with any fixed or mobile bearing component.

The inferior surface 20 of the tibial platform 14 includes a connecting spigot 24 and two pegs 26 projecting therefrom. The spigot 24 and pegs 26 are unitarily formed as part of the tibial platform 14.

The connecting spigot 24 includes a frusto-conical portion 28, which tapers outwardly in a direction towards the inferior surface 20 of the tibial platform 14 from its distal end. The frusto-conical portion 28 does not meet the inferior surface 20 of the tibial platform 14.

The taper of the frusto-conical portion 28 is, or is substantially, six degrees, but could be any other suitable angle which enables the formation of a required locking junction. The spigot 24 is positioned so that an anteriorally - posteriorally extending plane of symmetry 14a of the tibial platform 14 bisects or substantially bisects the spigot 24.

The pegs 26, which are smaller than the connecting spigot 24, are cylindrical or substantially cylindrical. The pegs 26 are positioned in spaced relationship symmetrically or substantially symmetrically about the connecting spigot 24, partway between the edge 30 of the tibial platform 14 and the anteriorally - posteriorally extending plane of symmetry.

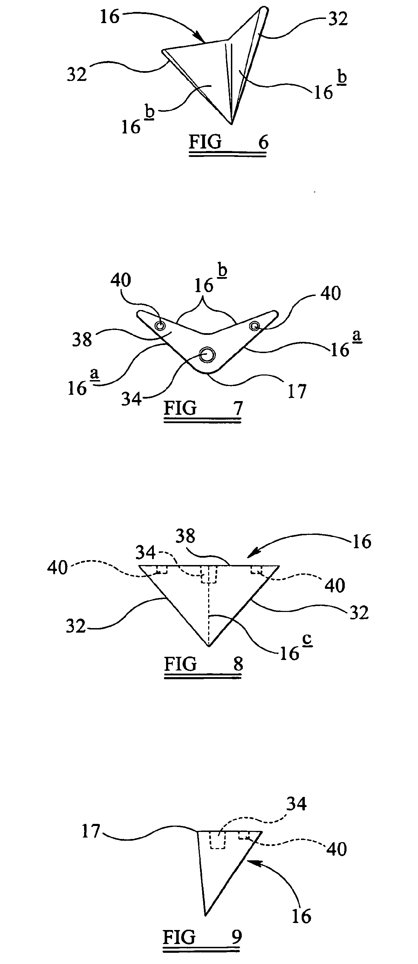

The keel 16 is generally wedge-shaped and provides a general impression of being a delta. In particular, the keel 16 is an inverted pyramid, but having a V-shaped or substantially V-shaped lateral cross-section, as can best be appreciated from Figures 6 and 7. A spine 17 of the keel 16 slopes to the vertex at or substantially at five degrees from the vertical. However, this may vary depending on the desired length of the keel 16.

By forming the keel 16 with two anterior sides 16a forming a generally convex arrangement and two posterior sides 16b forming a generally concave arrangement, an undercut can be achieved at an edge 32 of intersecting sides 16a, 16b when cutting along the plane of the sides 16a, 16b.

Furthermore, the keel 16 can be more easily accommodated beneath the generally kidney-shaped tibial platform 16, while still being comfortably received within the proximal tibial bone. This enables a significant area of contact to be maintained with the host bone without causing undue weakening, and thus allows suitable bone fixation of the keel.

An upper surface 38 of the keel 16 is provided with a substantially complementarily shaped socket 34 for receiving the connecting spigot 24. Similarly to the spigot 24, the socket 34 is positioned so that an anteriorally - posteriorally extending plane of symmetry 16c of the keel 16 bisects or substantially bisects the socket 34.

The socket 34 is frusto-conical shaped with a taper of, or substantially of, six degrees. Again, however, any suitable angle of taper can be utilised which permits the formation of a required locking junction. The depth of the socket 34 is such that the connecting spigot 24 of the tibial platform 14 does not abut the bottom surface 36 when received therein.

The upper surface 38 of the keel 16 is also provided with complementarily or substantially complementarily shaped peg openings 40 for receiving the pegs 26 of the tibial platform 14. The depth of the peg openings 40 is sufficient to prevent the pegs 26 from abutting the bottom surface 36 of the openings.

At least the inferior surface 20 of the tibial platform 14 includes a coating 42 of osteoconductive material, preferably in the form of plasma sprayed titanium and hydroxyapatite. The osteoconductive coating 42 covers the inferior surface 20.

The keel 16 can also include a coating 43 of the osteoconductive material, which, in this case, should at least be provided on the sides 16a and 16b. It is preferable that the osteoconductive material is not provided on the spine 17, the edges 32 and the upper surface 38 since this increases the difficulty of removal of the keel 16.

In use, once the proximal capsule of the tibia adjacent the distal end of the femur has been resected, the resected end of the tibia is reamed to accept the keel 16 of the tibial component 10 as a tight interference fit.

The tibial platform 14 is offered up to the keel 16 and the connecting spigot 24 and pegs 26 are introduced to the socket 34 and peg openings 40, respectively. The connecting spigot 24 is urged into the socket 34 until interference engagement of the tibial platform 14 and keel 16 is achieved. In this condition, there is a space 44 between the inferior surface 20 of the tibial platform 14 and the upper surface 38 of the keel 16, as best seen in Figures 1 and 2. Since the spigot 24 does not contact the bottom surface 36 of the socket 34, there is no play between the tibial platform 14 and the keel 16.

The assembled tibial component 10 is then offered up to the resected tibia, and the keel 16 is inserted so that the upper surface 38 is slightly recessed of the surrounding bone.

The space 44 between the inferior surface 20 of the tibial platform 14 and the upper surface 38 of the keel 16 is, or is substantially, 1.5 millimetre (mm) to 2 mm. However, it can be any suitable size of space providing a surgical saw blade can be accommodated therebetween.

Consequently, load imparted to the tibial platform 14 is transmitted directly to the tibia.

The pegs 26 of the tibial platform 14 are not load bearing and, when received in the peg openings 40, simply prevent or restrict rotation of the tibial platform 14 relative to the keel 16.

The osteoconductive material on the inferior surface 20 of the tibial component 10 and sides 16a, 16b of the keel 16 encourages apposition of host bone to the surfaces of the tibial component 10, and thus fixation of the tibial component 10 to the tibia.

Femoral, meniscal, fixed and mobile bearing components of a knee prosthesis incorporating the above-described tibial component 10 are well known and fitted in the normal manner. Consequently, detailed description will be omitted.

In the event of a revision being necessary, the tibial platform 14 can be removed from the keel 16. Since the inferior surface 20 of the tibial platform 14 is spaced from the upper surface 38 of the keel 16, channels 46 are generated between the pegs 26 and the connecting spigot 24 into which host bone can grow. It is thus a relatively straightforward matter to locate the space 44 between the tibial platform 14 and the keel 16 for the introduction of a surgical cutting implement. The space 44 between the tibial platform 14 and the keel 16 thus not only acts as a cutting guide, allowing the in-growth of host bone to be easily cut, but also enables resection from the anterior side of the posterior portion of the tibia which supports the platform.

Although typically unnecessary, the pegs 26 of the tibial platform 14 and/or the connecting spigot 24 can be cut. Since a greatly reduced, or no, synthetic material has to be removed in comparison with separation of a tibial platform from a standard tibial component, the amount of waste material introduced into the body as a result of the cutting action is greatly reduced, if not eliminated.

Once a sufficient amount of bone has been removed from under the tibial platform 14 and the channels 46 between the tibial platform 14 and the keel 16, a surgical osteotone can be inserted to lever the tibial platform 14 away from the keel 16.

With the upper surface 38 of the keel 16 exposed by the removal of the tibial platform 14, cuts which extend generally in the longitudinal direction of the tibia can be made along the sides 16a, 16b of the keel 16. Due to the pyramidical form of the keel 16, cuts parallel to the sides 16a, 16b of the keel 16 intersect, resulting in the keel 16 being undercut and thus easily removable from the tibia.

To enable pre-operative and intra-operative selection, along with post-operative revision, the tibial component can be provided as part of a modular tibial component system. The system comprises one or more sizes of the tibial platform and two or more sizes of the keel which are disengageably attachable to the or each tibial platform, as described above.

The tibial platforms and the keels are of various dimensions to suit different types of bone structures and to accommodate different situations. For example, a revision may be necessary to rectify a loose keel. In this case, a larger sized keel can be introduced without incurring extensive bone damage when trying to remove the original keel. New bone fixation can be achieved without necessarily resorting to long revision stems as is common practice.

It will be understood that, although the connecting spigot and pegs are described as being formed on the inferior surface of the tibial platform, and the socket and peg openings are described as being formed in the upper surface of the keel, the connecting spigot and/or the pegs can be formed on the keel, and the socket and/or the peg openings can be formed in the tibial platform.

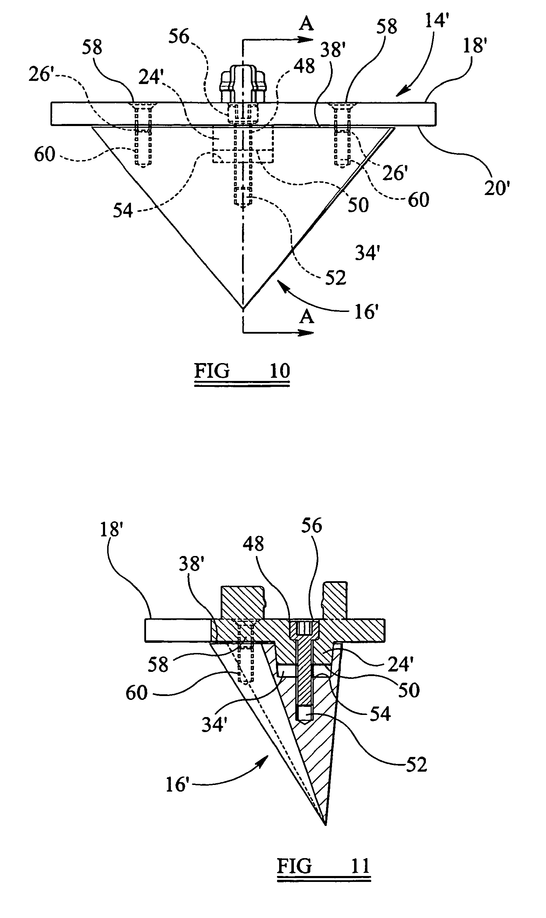

Referring to Figures 10 and 11, a second embodiment of a tibial component is shown. Like references refer to like parts, and further description will be omitted.

In this embodiment, connecting spigot 24' on inferior surface 20' of tibial platform 14' includes an axial through-hole 48 which opens out on superior surface 18' of the tibial platform 14' and distal end 50 of the spigot 24'. Socket 34', provided in the upper surface 38' of keel 16' and dimensioned as previously described for accepting the connecting spigot 24' as an interference fit, also includes a screw-threaded opening 52 in its bottom surface 54. A, typically anti-vibration, screw-threaded fastener 56 is thus be receivable in the axial through-hole 48 of the tibial platform 14' and is engageable in the opening 52 in the bottom 54 of the socket 34', thereby securely and releasably engaging the tibial platform 14' and the keel 16'. This arrangement minimises the risk of loosening occurring between the connecting spigot 24' and the socket 34'.

Further, typically anti-vibration, screw-threaded fasteners 58 are also receivable through anti-rotation pegs 26' formed on the inferior surface 20' of the tibial platform 14'. The fasteners 58 are releasably engageable in screw-threaded openings 60 formed in the bottom of peg openings 40' provided in the upper surface 38' of the keel 16'.

The screw-threaded fasteners 58 may be dispensed with in favour of only having the main screw-threaded fastener 56.

In this embodiment, when removing the tibial platform, the or each screw-threaded fastener is first released and removed using standard surgical tools. The procedure described above is then utilised to separate the tibial platform from the keel and tibia.

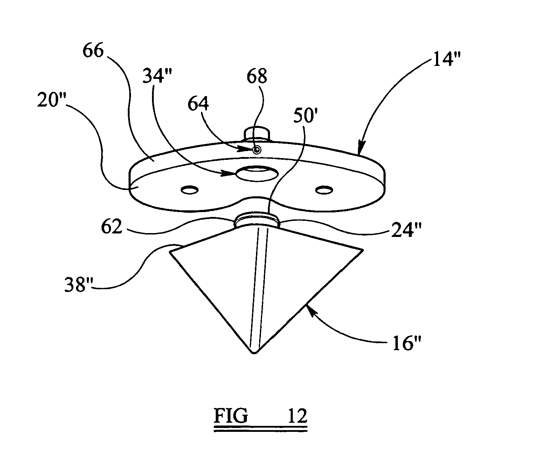

Referring to Figure 12, a third embodiment of a tibial component is shown. Again, like references refer to like parts, and further description will be omitted.

In this case, connecting spigot 24" is provided on upper surface 38" of keel 16", and socket 34", dimensioned as previously described for accepting the connecting spigot 24" as an interference fit, is provided in inferior surface 20" of tibial platform 14". The connecting spigot 24" is formed with a groove 62 adjacent to its distal end 50'. Preferably, the groove 62 is endless.

A through-hole 64 is formed through the anterior edge 66 of the tibial platform 14" and breaks out into the socket 34". The through-hole 64 is threaded to allow a, typically anti-vibration, screw threaded fastener 68 to be inserted into the tibial platform 14". When the connecting spigot 24" is received in the socket 34", the fastener 68 projects into the groove 62 of the connecting spigot 24", thus preventing separation of the tibial platform 14" and the keel 16" without first removing the fastener 68.

By providing the through-hole 64 on the anterior edge 66 of the tibial platform 14", access to the fastener 68 is simplified.

The tibial component described above is intended for use as part of a cementless knee prosthesis. However, the tibial component can be used as part of any type of knee prosthesis. In the situation where the keel is to be cemented in place, the coating of osteoconductive material is typically dispensed with.

Although the keel is pyramid shaped, other types of polyhedron may also be suitable by allowing the necessary undercut.

All exterior surfaces of the keel can include the coating of osteoconductive material.

The osteoconductive coating on the inferior surface of the tibial platform may only cover a portion of the inferior surface of the tibial component. For example, the osteoconductive coating may form an outline of the shape of the upper surface of the keel, instead of covering the entire inferior surface. In this case, the osteoconductive coating is provided between the perimeter edge of the inferior surface of the tibial platform and the outline shape of the upper surface of the keel.

One or more than two anti-rotation pegs can be provided. A matching number of peg openings are thus provided.

## Claims

1. A tibial component for a knee prosthesis (12), the tibial component (10) comprising a tibial platform (14;14';14") and a wedge-shaped keel (16;16';16") which is disengageably attachable to the tibial platform (14;14';14"), the tibial platform (14;14';14") having a superior surface (18;18') for supporting a fixed or mobile bearing component and an inferior surface (20;20';20") which, when the tibial platform (14;14';14") and the keel (16;16';16") are engaged with each other, defines a space (44) between it and an upper surface (38;38';38") of the keel (16;16';16")**characterised in that** the keel includes two contiguous anterior sides (16a) and two contiguous posterior sides (16b), the anterior and posterior sides (16a, 16b) converging at a vertex of the keel and defining a V-shaped lateral cross-section along an extent from the upper surface (38; 38'; 38") of the keel to the vertex, the keel (38; 38'; 38') thus being completely undercuttable by cuts parallel to the said sides (16a, 16b), and the space (44) being a cutting guide dimensioned for receiving a surgical saw blade.

2. A tibial component as claimed in claim 1, wherein one or more channels (46) are defined in the space (44) between the inferior surface (20;20';20") of the tibial platform (14;14';14") and the upper surface (38;38';38") of the keel (16;16';16").

3. A tibial component as claimed in claim 1 or claim 2, wherein the inferior surface (20;20';20") of the tibial platform (14;14';14") includes a coating of osteoconductive material.

4. A tibial component as claimed in any one of the preceding claims, wherein the superior surface (18;18') of the tibial platform (14;14';14") is adapted to support a totally mobile meniscal component.

5. A tibial component as claimed in any one of the preceding claims, wherein at least a portion of the keel (16;16';16") includes a coating of osteoconductive material.

6. A tibial component as claimed in any one of the preceding claims, further comprising a connecting spigot (24;24';24") provided on the inferior surface (20;20';20") of the tibial platform (14;14';14") or an upper surface (38;38';38") of the keel (16;16';16"), and a substantially complementarily shaped socket (34;34';34") provided in the upper surface (38;38';38") of the keel (16;16';16") or the inferior surface (20;20';20") of the tibial platform (14;14';14"), the connecting spigot (24;24';24") being receivable in the socket (34;34';34") to engage the tibial platform (14;14';14") and the keel (16;16';16").

7. A tibial component as claimed in claim 6, wherein the connecting spigot (24;24';24") is dimensioned to be an interference fit in the socket (34;34';34") by which the inferior surface (20;20';20") of the tibial platform (14;14';14") is held in spaced (44) relationship with the upper surface (38;38';38") of the keel (16;16';16").

8. A tibial component as claimed in claim 6 or claim 7, wherein the connecting spigot (24;24';24") and the socket (34;34';34") include mating frusto-conical portions.

9. A tibial component as claimed in any one of claims 6 to 8, wherein the connecting spigot (24;24';24") is positioned in or substantially in a plane of symmetry of the tibial platform (14;14';14") or the keel (16;16';16").

10. A tibial component as claimed in claim 6 to 9, further comprising an anti-vibration screw-threaded fastener (56,58;68) for fastening the tibial platform (14';14") to the keel (16';16").

11. A tibial component as claimed in claim 10, wherein the screw-threaded fastener (56) is receivable through the spigot (24') and the socket (34').

12. A tibial component as claimed in claim 10, wherein the screw-threaded fastener (68) is receivable through an edge (66) of the tibial platform (14") and is engageable with the connecting spigot (24").

13. A tibial component as claimed in any one of claims 6 to 12, further comprising one or more pegs (26;26') provided on the inferior surface (20;20';20") of the tibial platform (14;14';14") or an upper surface (38;38';38") of the keel (16;16';16"), and a complementarily shaped peg opening (40;40') provided in the upper surface (38;38';38") of the keel (16;16';16") or the inferior surface (20;20';20") of the tibial platform (14;14';14"), the or each peg (26;26') being receivable in the respective peg opening (40;40') to prevent or limit relative rotation of the tibial platform (14;14';14") and the keel (16;16';16").

14. A tibial component as claimed in claim 13, wherein two said pegs (26;26') are provided, each peg (26;26') being positioned between the connecting spigot (24;24';24") or the socket (34;34';34") and an edge (30) of the tibial platform (14;14';14") or the keel (16;16';16").

15. A tibial component as claimed in any one of the preceding claims, wherein the keel (16;16';16") is delta shaped.

16. A tibial component as claimed in any one of the preceding claims, wherein the keel (16;16';16") is a polyhedron.

17. A modular tibial component system for a knee prosthesis, the system comprising one or more tibial components as claimed in any one of the preceding claims, wherein two or more said wedge-shaped keels (16;16';16") are provided, said keels (16,16',16") being disengageably attachable to one or more said tibial platforms (14;14';14"), and the tibial platforms (14;14';14") and keels (16;16';16") varying in dimensions, wherein each tibial platform (14;14';14") and keel (16;16';16") is selectable intraoperatively.

## Patentansprüche

1. Tibiale Komponente für eine Knieprothese (12), wobei die tibiale Komponente (10) eine tibiale Plattform (14; 14'; 14") und einen keilförmigen Kiel (16; 16'; 16") umfasst, der außer Eingriff bringbar an die tibiale Plattform (14; 14'; 14") anfügbar ist, wobei die tibiale Plattform (14; 14'; 14") eine obere Oberfläche (18; 18') zum Tragen einer festen oder beweglichen Lagerkomponente und eine untere Oberfläche (20; 20'; 20") aufweist, die, wenn die tibiale Plattform (14; 14'; 14") und der Kiel (16; 16'; 16") miteinander in Eingriff sind, einen Raum (44) dazwischen und einer oberen Oberfläche (38; 38'; 38") des Kiels (16; 16'; 16") definiert, **dadurch gekennzeichnet, dass** der Kiel zwei zusammenhängende Vorderseiten (16a) und zwei zusammenhängende Rückseiten (16b) umfasst, wobei die Vorder- und Rückseiten (16a, 16b) an einem Scheitel des Kiels konvergieren und einen V-förmigen lateralen Querschnitt entlang einer Größe von der oberen Oberfläche (38; 38'; 38") des Kiels zum Scheitel definieren, wobei der Kiel (38; 38'; 38") folglich völlig durch Schnitte parallel zu den Seiten (16a, 16b) unterschneidbar ist und der Raum (44) eine Schnittführung ist, die zum Aufnehmen eines chirurgischen Sägeblatts dimensioniert ist.

2. Tibiale Komponente nach Anspruch 1, wobei einer oder mehrere Kanäle (46) im Raum (44) zwischen der unteren Oberfläche (20; 20'; 20") der tibialen Plattform (14; 14'; 14") und der oberen Oberfläche (38; 38'; 38") des Kiels (16; 16'; 16") definiert sind.

3. Tibiale Komponente nach Anspruch 1 oder Anspruch 2, wobei die untere Oberfläche (20; 20'; 20") der tibialen Plattform (14; 14'; 14") eine Beschichtung aus osteoleitfähigem Material umfasst.

4. Tibiale Komponente nach einem der vorhergehenden Ansprüche, wobei die obere Oberfläche (18; 18') der tibialen Plattform (14; 14'; 14") angepasst ist, eine völlig bewegliche meniskenförmige Komponente zu tragen.

5. Tibiale Komponente nach einem der vorhergehenden Ansprüche, wobei mindestens ein Teil des Kiels (16; 16'; 16") eine Beschichtung aus osteoleitfähigem Material umfasst.

6. Tibiale Komponente nach einem der vorhergehenden Ansprüche, die weiter einen Verbindungszapfen (24; 24'; 24") umfasst, der auf der unteren Oberfläche (20; 20'; 20") der tibialen Plattform (14; 14'; 14") oder einer oberen Oberfläche (38; 38'; 38") des Kiels (16; 16'; 16") bereitgestellt ist und eine im Wesentlichen komplementär geformte Muffe (34; 34'; 34") in der oberen Oberfläche (38; 38'; 38") des Kiels (16; 16'; 16") oder der unteren Oberfläche (20; 20'; 20") der tibialen Plattform (14; 14'; 14") bereitgestellt ist, wobei der Verbindungszapfen (24; 24'; 24") in der Muffe (34; 34'; 34") aufnehmbar ist, um die tibiale Plattform (14; 14'; 14") und den Kiel (16; 16'; 16") in Eingriff zu bringen.

7. Tibiale Komponente nach Anspruch 6, wobei der Verbindungszapfen (24; 24'; 24") dimensioniert ist, eine Presspassung in der Muffe (34; 34'; 34") zu sein, wodurch die untere Oberfläche (20; 20'; 20") der tibialen Plattform (14; 14'; 14") in räumlicher (44) Beziehung zur oberen Oberfläche (38; 38'; 38") des Kiels (16; 16'; 16") gehalten wird.

8. Tibiale Komponente nach Anspruch 6 oder Anspruch 7, wobei der Verbindungszapfen (24; 24'; 24") und die Muffe (34; 34'; 34") zusammenpassende kegelstumpfförmige Teilstücke umfassen.

9. Tibiale Komponente nach einem der Ansprüche 6 bis 8, wobei der Verbindungszapfen (24; 24'; 24") in einer Symmetrieebene der tibialen Plattform (14; 14'; 14") oder des Kiels (16; 16'; 16") positioniert oder im Wesentlichen positioniert ist.

10. Tibiale Komponente nach Anspruch 6 bis 9, die weiter ein vibrationsgeschütztes Schraubengewindebefestigungselement (56, 58; 68) zum Befestigen der tibialen Plattform (14'; 14") am Kiel (16 ; 16") umfasst.

11. Tibiale Komponente nach Anspruch 10, wobei das Schraubengewindebefestigungselement (56) durch den Zapfen (24') und die Muffe (34') aufnehmbar ist.

12. Tibiale Komponente nach Anspruch 10, wobei das Schraubengewindebefestigungselement (68) durch eine Kante (66) der tibialen Plattform (14") aufnehmbar ist und mit dem Verbindungszapfen in Eingriff bringbar ist.

13. Tibiale Komponente nach einem der Ansprüche 6 bis 12, die weiter einen oder mehrere Dübel (26; 26'; 26") umfasst, die auf der unteren Oberfläche (20; 20'; 20") der tibialen Plattform (14; 14'; 14'') oder einer oberen Oberfläche (38; 38'; 38") des Kiels (16; 16'; 16") bereitgestellt sind und eine komplementäre Dübelöffnung (40; 40') in der oberen Oberfläche (38; 38'; 38") des Kiels (16; 16'; 16") oder der unteren Oberfläche (20; 20'; 20") der tibialen Plattform (14; 14'; 14") bereitgestellt ist, wobei der oder jeder Dübel (26; 26') in der Dübelöffnung (40; 40') aufnehmbar ist, um relative Drehung der tibialen Plattform (14; 14'; 14") und des Kiels (16; 16'; 16") zu verhindern oder zu begrenzen.

14. Tibiale Komponente nach Anspruch 13, wobei zwei Dübel (26; 26') bereitgestellt sind und jeder Dübel (26; 26') zwischen dem Verbindungszapfen (24; 24'; 24") oder der Muffe (34; 34'; 34") und einer Kante (30) der tibialen Plattform (14; 14'; 14") oder des Kiels (16; 16'; 16") positioniert ist.

15. Tibiale Komponente nach einem der vorhergehenden Ansprüche, wobei der Kiel (16; 16'; 16") deltaförmig ist.

16. Tibiale Komponente nach einem der vorhergehenden Ansprüche, wobei der Kiel (16; 16'; 16") ein Polyeder ist.

17. Modulares tibiales Komponentensystem für eine Knieprothese, wobei das System eine oder mehrere tibiale Komponenten nach einem der vorhergehenden Ansprüche umfasst, wobei zwei oder mehrere der keilförmigen Kiele (16; 16'; 16") bereitgestellt sind, die Kiele (16; 16'; 16") außer Eingriff bringbar an einer oder mehreren der tibialen Plattformen (14; 14'; 14") anfügbar sind und die tibialen Plattformen (14; 14'; 14") und Kiele (16; 16; 16") in den Dimensionen variieren, wobei jede tibiale Plattform (14; 14'; 14") und jeder Kiel (16; 16 ; 16") intraoperativ wählbar ist.

## Revendications

1. Composant tibial pour une prothèse de genou (12), le composant tibial (10) comprenant une plate-forme tibiale (14; 14'; 14") et une quille en forme de coin (16 ; 16' ; 16"), pouvant être fixée sur la plate-forme tibiale (14 ; 14' ; 14") de sorte à pouvoir en être dégagée, la plate-forme tibiale (14 ; 14' ; 14") comportant une surface supérieure (18 ; 18') pour supporter un composant de support fixe ou mobile, et une surface inférieure (20 ; 20' ; 20"), définissant, lors de l'engagement mutuel de la plate-forme tibiale (14 ; 14' ; 14") et de la quille (16 ; 16' ; 16"), un espace (44) entre elle et une surface supérieure (38 ; 38' ; 38") de la quille (16 ; 16' ; 16"), **caractérisé en ce que** la quille englobe deux côtés antérieurs contigus (16a) et deux côtés postérieurs continus (16b), les côtés antérieurs et postérieurs (16a, 16b) convergeant au niveau d'un sommet de la quille et définissant une section transversale latérale en forme de V le long d'une extension s'étendant de la surface supérieure (38 ; 38' ; 38") de la quille vers le sommet, la quille (38 ; 38' ; 38") pouvant ainsi être découpée complètement par des coupes parallèles aux dits côtés (16a, 16b) et l'espace (44) constituant un guide de coupe dimensionné de sorte à recevoir une lame de scie chirurgicale.

2. Composant tibial selon la revendication 1, dans lequel un ou plusieurs canaux (46) sont définis dans l'espace (44) entre la surface inférieure (20 ; 20'; 20") de la plate-forme tibiale (14 ; 14'; 14") et la surface supérieure (38 ; 38' ; 38") de la quille (16 ; 16'; 16").

3. Composant tibial selon les revendications 1 ou 2, dans lequel la surface inférieure (20 ; 20' ; 20") de la plate-forme tibiale (14 ; 14' ; 14") englobe un revêtement de matériau ostéoconducteur.

4. Composant tibial selon l'une quelconque des revendications précédentes, dans lequel la surface supérieure (18 ; 18') de la plate-forme tibiale (14 ; 14' ; 14") est adaptée pour supporter un composant méniscal à mobilité totale.

5. Composant tibial selon l'une quelconque des revendications précédentes, dans lequel au moins une partie de la quille (16 ; 16' ; 16") englobe un revêtement de matériau ostéoconducteur.

6. Composant tibial selon l'une quelconque des revendications précédentes, comprenant en outre un tourillon de raccordement (24 ; 24' ; 24") agencé sur la surface inférieure (20 ; 20' ; 20") de la plate-forme tibiale (14 ; 14' ; 14") ou sur une surface supérieure (38 ; 38' ; 38") de la quille (16 ; 16' ; 16"), et une douille de forme pratiquement complémentaire (34 ; 34' ; 34") agencée dans une surface supérieure (38 ; 38' ; 38") de la quille (16 ; 16' ; 16") ou la surface inférieure (20 ; 20' ; 20") de la plate-forme tibiale (14 ; 14' ; 14"), le tourillon de raccordement (24 ; 24' ; 24") pouvant être reçu dans la douille (34 ; 34' ; 34") pour s'engager dans la plate-forme tibiale (14 ; 14' ; 14") et la quille (16 ; 16' ; 16").

7. Composant tibial selon la revendication 6, dans lequel le tourillon de raccordement (24 ; 24' ; 24") est dimensionné de sorte à permettre un ajustement serré dans la douille (34 ; 24' ; 34"), la surface inférieure (20 ; 20' ; 20") de la plate-forme tibiale (14 ; 14' ; 14") étant ainsi maintenue dans une relation espacée (44) par rapport à la surface supérieure (38 ; 38' ; 38") de la quille (16 ; 16' ; 16").

8. Composant tibial selon les revendications 6 ou 7, dans lequel le tourillon de raccordement (24 ; 24' ; 24") et la douille (34 ; 34' ; 34") englobent des parties en tronc de cône complémentaires.

9. Composant tibial selon l'une quelconque des revendications 6 à 8, dans lequel le tourillon de raccordement (24 ; 24' ; 24") est positionné ou pratiquement positionné dans un plan de symétrie de la plate-forme tibiale (14; 14' ; 14") ou de la quille (16 ; 16' ; 16").

10. Composant tibial selon les revendications 6 à 9, comprenant en outre un élément de fixation anti-vibratoire à filet de vis (56, 58 ; 68) pour fixer la plate-forme tibiale (14' ; 14") sur la quille (16' ; 16").

11. Composant tibial selon la revendication 10, dans lequel l'élément de fixation à filet de vis (56) peut être reçu à travers le tourillon (24') et la douille (34').

12. Composant tibial selon la revendication 10, dans lequel l'élément de fixation à filet de vis (68) peut être reçu à travers un bord (66) de la plate-forme tibiale (14") et peut s'engager dans le tourillon de raccordement (24").

13. Composant tibial selon l'une quelconque des revendications 6 à 12, comprenant en outre une ou plusieurs chevilles (26 ; 26') agencées sur la surface inférieure (20 ; 20' ; 20") de la plate-forme tibiale (14 ; 14' ; 14") ou une surface supérieure (38 ; 38' ; 38") de la quille (16 ; 16' ; 16"), et une ouverture de cheville de forme complémentaire (40 ; 40') formée dans la surface supérieure (38 ; 38' ; 38") de la quille (16 ; 16' ; 16") ou la surface inférieure (20, 20' ; 20") de la plate-forme tibiale (14 ; 14' ; 14"), la ou chaque cheville (26 ; 26') pouvant être reçue dans l'ouverture de cheville respective (40 ; 40') pour empêcher ou limiter une rotation relative entre la plate-forme tibiale (14 ; 14' ; 14") et la quille (16 ; 16' ; 16").

14. Composant tibial selon la revendication 13, comportant deux dites chevilles (26 ; 26'), chaque cheville (26 ; 26') étant positionnée entre le tourillon de raccordement (24 ; 24' ; 24") ou la douille (34 ; 34' ; 34") et un bord (30) de la plate-forme tibiale (14 ; 14' ; 14") ou la quille (16 ; 16' ; 16").

15. Composant tibial selon l'une quelconque des revendications précédentes, dans lequel la quille (16 ; 16' ; 16") a une forme en delta.

16. Composant tibial selon l'une quelconque des revendications précédentes, dans lequel la quille (16 ; 16' ; 16") est constituée par un polyèdre.

17. Système de composant tibial modulaire pour une prothèse de genou, le système comprenant un ou plusieurs composants tibiaux selon l'une quelconque des revendications précédentes, comportant deux ou plusieurs quilles en forme de coin (16 ; 16' ; 16"), lesdites quilles (16 ; 16' ; 16") pouvant être fixées sur une ou plusieurs desdites plates-formes tibiales (14 ; 14' ; 14") et être dégagées de celles-ci, les plates-formes tibiales (14 ; 14' ; 14") et les quilles (16 ; 16' ; 16") ayant des dimensions variables, chaque plate-forme tibiale (14; 14' ; 14") et chaque quille (16 ; 16' ; 16") pouvant être sélectionnées en cours d'opération.
